# EUROPEAN PATENT SPECIFICATION

(11) **EP 3 518 776 B1**
(45) Date of publication and mention of the grant of the patent: **25.11.2020**
(21) Application number: 17780657.7
(22) Date of filing: 22.09.2017
(51) Int. Cl.: A61B 8/12, A61B 8/00, B06B 1/06

(54) **GUIDE MEMBER FOR ELECTRICAL CABLE ALIGNMENT AND ATTACHMENT AND ASSOCIATED INTRALUMINAL DEVICES**
FÜHRUNGSELEMENT FÜR DIE AUSRICHTUNG UND BEFESTIGUNG ELEKTRISCHER KABEL UND ZUGEHÖRIGE INTRALUMINALE VORRICHTUNGEN
ÉLÉMENT DE GUIDAGE POUR ALIGNEMENT ET FIXATION DE CÂBLE ÉLECTRIQUE AINSI QUE DISPOSITIFS

(30) Priority: 29.09.2016 US 201662401671 P
(43) Date of publication of application: 07.08.2019
(73) Proprietor: Koninklijke Philips N.V., 5656 AG Eindhoven (NL)
(72) Inventor: WROLSTAD, David, Kenneth, 5656 AE Eindhoven (NL)
(74) Representative: Philips Intellectual Property & Standards
(86) International application number: PCT/EP2017/073970
(87) International publication number: WO 2018/060061

(56) References cited:
- GB-A- 2 315 020
- US-A- 5 947 905
- US-A1- 2016 007 962

## Description

### TECHNICAL FIELD

The present disclosure relates generally to intraluminal imaging and, in particular, to an imaging assembly of an intraluminal imaging device. For example, the imaging assembly can include guide member through which wires of an electrical cable extend. The guide member aligns the wires to corresponding conductive pads of a flex circuit and is affixed to the flex circuit. The guide member improves manufacturing efficiency and strengthens mechanical attachment of the electrical cable and the imaging assembly.

### BACKGROUND

Intravascular ultrasound (IVUS) imaging is widely used in interventional cardiology as a diagnostic tool for assessing a diseased vessel, such as an artery, within the human body to determine the need for treatment, to guide the intervention, and/or to assess its effectiveness. An IVUS device including one or more ultrasound transducers is passed into the vessel and guided to the area to be imaged. The transducers emit ultrasonic energy in order to create an image of the vessel of interest. Ultrasonic waves are partially reflected by discontinuities arising from tissue structures (such as the various layers of the vessel wall), red blood cells, and other features of interest. Echoes from the reflected waves are received by the transducer and passed along to an IVUS imaging system. The imaging system processes the received ultrasound echoes to produce a cross-sectional image of the vessel where the device is placed.

Solid-state (also known as synthetic-aperture) IVUS catheters are one of the two types of IVUS devices commonly used today, the other type being the rotational IVUS catheter. Solid-state IVUS catheters carry a scanner assembly that includes an array of ultrasound transducers distributed around its circumference along with one or more integrated circuit controller chips mounted adjacent to the transducer array. The controllers select individual transducer elements (or groups of elements) for transmitting an ultrasound pulse and for receiving the ultrasound echo signal. By stepping through a sequence of transmit-receive pairs, the solid-state IVUS system can synthesize the effect of a mechanically scanned ultrasound transducer but without moving parts (hence the solid-state designation). Since there is no rotating mechanical element, the transducer array can be placed in direct contact with the blood and vessel tissue with minimal risk of vessel trauma. Furthermore, because there is no rotating element, the electrical interface is simplified. The solid-state scanner can be wired directly to the imaging system with a simple electrical cable and a standard detachable electrical connector, rather than the complex rotating electrical interface required for a rotational IVUS device.

The electrical cable and the solid-state scanner are connected during assembly of the IVUS device. Generally, this requires that conductors in the electrical cable be individually aligned with a respective conductive pad on the solid-state scanner. The electrical cable includes many different conductors. Accordingly, individually aligning the conductors and the conductive pads can be a time-consuming step during manufacturing.

The conductors are usually soldered to the conductive pads, which electrically couples the electrical couples the electrical cable and the solid-state scanner. Soldering also mechanically attaches the electrical cable and the solid-state scanner. While attachment of the conductors to the conductive pads is often the only mechanical connection between the electrical cable and the solid-state scanner, the mechanical connection is not strong. During manufacturing of the IVUS device, the electrical cable and the solid-state scanner are variously grasped and moved. Inadvertent handling of the electrical cable and the solid-state scanner, such as pulling on the electrical cable, can detach the conductors from the conductive pads. The solid-state scanner may need to be discarded as a result, which results in increased waste and cost, or the conductors must again be individually reattached, which increases the manufacturing time.

Closest prior art document US 2016/007962 A1 discloses an intraluminal imaging device, comprising:
a. a flexible elongate member configured for positioning within a vessel of a patient, the flexible elongate member including a proximal portion and a distal portion;
b. an imaging assembly disposed at the distal portion of the flexible elongate member, the imaging assembly including a conductor interface;
c. a plurality of wires extending along a length of the flexible elongate member and in communication with the imaging assembly, wherein the plurality of wires are coupled to the imaging assembly at the conductor interface; and
d. a guide member disposed adjacent to the conductor interface, wherein the plurality of wires extend through the guide member and wherein the guide member collectively aligns the plurality of wires to the conductor interface.

The document also discloses a method of assembling an intraluminal imaging device, the method comprising:
a. obtaining an imaging assembly including a conductor interface;
b. obtaining a plurality of wires extending through a guide member;
c. collectively aligning the plurality of wires to the conductor interface using the guide member; and
d. coupling the plurality of wires to the imaging assembly at the conductor interface. The guide member is connected to the conductor interface by means of a mechanical snap-fit mechanism.

### SUMMARY

The invention provides imaging devices, systems, and related methods that overcome the limitations associated with individually aligning and soldering conductors of imaging assemblies.

Embodiments of the present disclosure provide an improved intraluminal ultrasound imaging device for generating images of a blood vessel. A guide member is positioned around the wires forming an electrical cable at a distal portion of the electrical cable. During assembly, the wires can be aligned with a respective conductive pad of an imaging assembly by aligning the guide member to the imaging assembly. Manufacturing efficiency is improved by aligning the guide member as a whole to the imaging assembly rather than individually aligning each of the wires and conductive pads. The guide member can also be attached to the imaging assembly, such as by an adhesive. Accordingly, a more robust mechanical connection is formed between which the electrical cable and the imaging assembly.

In one embodiment, an intraluminal imaging device : according to claim 1 is provided.

Further aspects are defined in dependent claims 2-12.

In one embodiment, a method of assembling an intraluminal imaging device according to claim 13 is provided.

Further aspects are defined in dependent claims 14 and 15.

Additional aspects, features, and advantages of the present disclosure will become apparent from the following detailed description.

### BRIEF DESCRIPTION OF THE DRAWINGS

Illustrative embodiments of the present disclosure will be described with reference to the accompanying drawings, of which:
Fig. 1 is a diagrammatic schematic view of an imaging system, according to aspects of the present disclosure.
Fig. 2 is a diagrammatic top view of a scanner assembly in a flat configuration, according to aspects of the present disclosure.
Fig. 3 is a diagrammatic side view of a scanner assembly in a rolled configuration around a support member, according to aspects of the present disclosure.
Fig. 4 is a diagrammatic cross-sectional side view of a distal portion of an intraluminal device, according to aspects of the present disclosure.
Fig. 5 is a diagrammatic perspective view of a flex circuit coupled to an electrical cable, according to aspects of the present disclosure.
Fig. 6 is a diagrammatic perspective view of a flex circuit coupled to an electrical cable, according to aspects of the present disclosure.
Fig. 7 is a diagrammatic top view of a flex circuit coupled to an electrical cable, according to aspects of the present disclosure.
Fig. 8A is a diagrammatic top view of a guide member, according to aspects of the present disclosure.
Fig. 8B is a diagrammatic bottom view of the guide member of Fig. 8A, according to aspects of the present disclosure.
Fig. 8C is a diagrammatic back view of the guide member of Fig. 8A, according to aspects of the present disclosure.
Fig. 8D is a diagrammatic side view of the guide member of Fig. 8A, according to aspects of the present disclosure.
Fig. 9 is a diagrammatic perspective view of a guide member and an electrical cable, according to aspects of the present disclosure.
Fig. 10 is a diagrammatic perspective view of a scanner assembly coupled to an electrical cable, according to aspects of the present disclosure.
Fig. 11 is a diagrammatic top view of a scanner assembly coupled to an electrical cable, according to aspects of the present disclosure.
Fig. 12 is a diagrammatic side view of a scanner assembly coupled to an electrical cable, according to aspects of the present disclosure.
Fig. 13 is a diagrammatic back view of a guide member, according to aspects of the present disclosure.
Fig. 14 is a flow diagram of a method of assembly an intraluminal imaging device, according to aspects of the present disclosure.

### DETAILED DESCRIPTION

For the purposes of promoting an understanding of the principles of the present disclosure, reference will now be made to the embodiments illustrated in the drawings, and specific language will be used to describe the same. It is nevertheless understood that no limitation to the scope of the disclosure is intended. Any alterations and further modifications to the described devices, systems, and methods, and any further application of the principles of the present disclosure are fully contemplated and included within the present disclosure as would normally occur to one skilled in the art to which the disclosure relates. In particular, it is fully contemplated that the features, components, and/or steps described with respect to one embodiment may be combined with the features, components, and/or steps described with respect to other embodiments of the present disclosure. For the sake of brevity, however, the numerous iterations of these combinations will not be described separately.

The present disclosure describes an imaging assembly for an intraluminal imaging device. The imaging assembly includes a flex circuit positioned at a distal portion of a flexible elongate member. A conductor interface having conductive pads extends from a proximal portion of the flex circuit. Wires of an electrical cable are coupled to respective conductive pads of the imaging assembly at the conductor interface. The wires extend through respective bores of a guide member that aligns the wires with respect to the conductor interface. The guide member is positioned at a distal portion of the electrical cable. The guide member can be coupled to the conductor interface to mechanically couple the electrical cable and the imaging assembly.

The intraluminal imaging device described herein achieves numerous advantages. For example, the guide member through the wires extend facilitates a faster, less labor-intensive, and more efficient manufacturing process for the intraluminal device. In that regard, rather than individually aligning the wires to the respective conductive pads, all of the wires can be collectively aligned with the conductive pads when the guide member is aligned to the conductor interface. Additionally, coupling the guide member to the conductor interface creates a more robust imaging assembly. For example, joining surfaces of the guide member and the conductor interface via an adhesive creates a connection that can be more resilient than solder/weld interface between wires and the conductive pads.

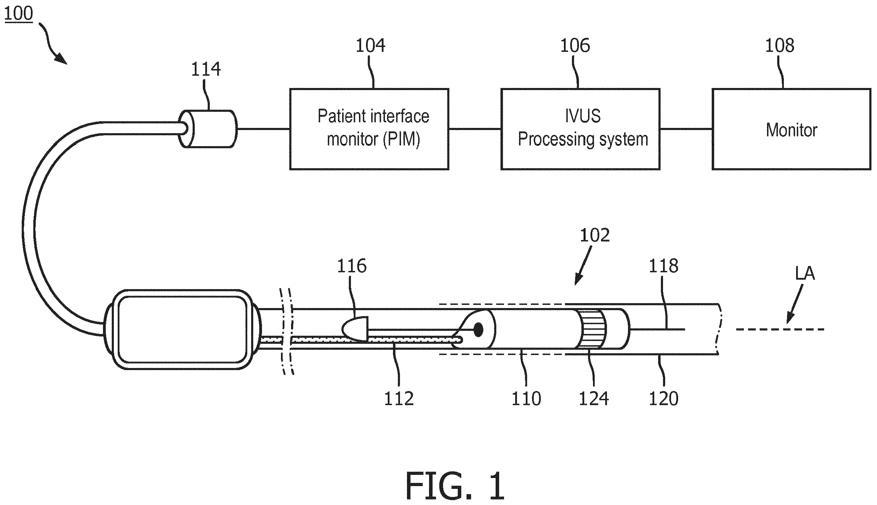

Fig. 1 is a diagrammatic schematic view of an intravascular ultrasound (IVUS) imaging system 100, according to aspects of the present disclosure. The IVUS imaging system 100 may include a solid-state IVUS device 102 such as a catheter, guide wire, or guide catheter, a patient interface module (PIM) 104, an IVUS processing system or console 106, and a monitor 108.

At a high level, the IVUS device 102 emits ultrasonic energy from a transducer array 124 included in scanner assembly 110 mounted near a distal end of the catheter device. The ultrasonic energy is reflected by tissue structures in the medium, such as a vessel 120, surrounding the scanner assembly 110, and the ultrasound echo signals are received by the transducer array 124. The PIM 104 transfers the received echo signals to the console or computer 106 where the ultrasound image (including the flow information) is reconstructed and displayed on the monitor 108. The console or computer 106 can include a processor and a memory. The computer or computing device 106 can be operable to facilitate the features of the IVUS imaging system 100 described herein. For example, the processor can execute computer readable instructions stored on the non-transitory tangible computer readable medium.

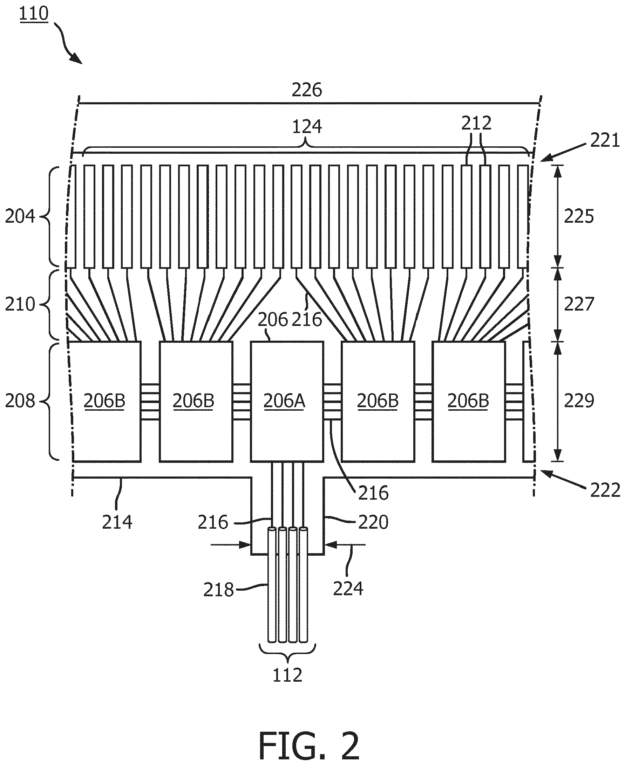

The PIM 104 facilitates communication of signals between the IVUS console 106 and the scanner assembly 110 included in the IVUS device 102. This communication includes the steps of: (1) providing commands to integrated circuit controller chip(s) 206A, 206B, illustrated in Fig. 2, included in the scanner assembly 110 to select the particular transducer array element(s) to be used for transmit and receive, (2) providing the transmit trigger signals to the integrated circuit controller chip(s) 206A, 206B included in the scanner assembly 110 to activate the transmitter circuitry to generate an electrical pulse to excite the selected transducer array element(s), and/or (3) accepting amplified echo signals received from the selected transducer array element(s) via amplifiers included on the integrated circuit controller chip(s) 126 of the scanner assembly 110. In some embodiments, the PIM 104 performs preliminary processing of the echo data prior to relaying the data to the console 106. In examples of such embodiments, the PIM 104 performs amplification, filtering, and/or aggregating of the data. In an embodiment, the PIM 104 also supplies high- and low-voltage DC power to support operation of the device 102 including circuitry within the scanner assembly 110.

The IVUS console 106 receives the echo data from the scanner assembly 110 by way of the PIM 104 and processes the data to reconstruct an image of the tissue structures in the medium surrounding the scanner assembly 110. The console 106 outputs image data such that an image of the vessel 120, such as a cross-sectional image of the vessel 120, is displayed on the monitor 108. Vessel 120 may represent fluid filled or surrounded structures, both natural and man-made. The vessel 120 may be within a body of a patient. The vessel 120 may be a blood vessel, as an artery or a vein of a patient's vascular system, including cardiac vasculature, peripheral vasculature, neural vasculature, renal vasculature, and/or or any other suitable lumen inside the body. For example, the device 102 may be used to examine any number of anatomical locations and tissue types, including without limitation, organs including the liver, heart, kidneys, gall bladder, pancreas, lungs; ducts; intestines; nervous system structures including the brain, dural sac, spinal cord and peripheral nerves; the urinary tract; as well as valves within the blood, chambers or other parts of the heart, and/or other systems of the body. In addition to natural structures, the device 102 may be may be used to examine man-made structures such as, but without limitation, heart valves, stents, shunts, filters and other devices.

In some embodiments, the IVUS device includes some features similar to traditional solid-state IVUS catheters, such as the EagleEye® catheter available from Volcano Corporation and those disclosed in U.S. Patent No. 7,846,101. For example, the IVUS device 102 includes the scanner assembly 110 near a distal end of the device 102 and a transmission line bundle 112 extending along the longitudinal body of the device 102. The transmission line bundle or cable 112 can include a plurality of conductors, including one, two, three, four, five, six, seven, or more conductors 218 (Fig. 2). It is understood that any suitable gauge wire can be used for the conductors 218. In an embodiment, the cable 112 can include a four-conductor transmission line arrangement with, e.g., 41 AWG gauge wires. In an embodiment, the cable 112 can include a seven-conductor transmission line arrangement utilizing, e.g., 44 AWG gauge wires. In some embodiments, 43 AWG gauge wires can be used.

The transmission line bundle 112 terminates in a PIM connector 114 at a proximal end of the device 102. The PIM connector 114 electrically couples the transmission line bundle 112 to the PIM 104 and physically couples the IVUS device 102 to the PIM 104. In an embodiment, the IVUS device 102 further includes a guide wire exit port 116. Accordingly, in some instances the IVUS device is a rapid-exchange catheter. The guide wire exit port 116 allows a guide wire 118 to be inserted towards the distal end in order to direct the device 102 through the vessel 120.

The IVUS device 102 includes a flexible elongate member 115 having a proximal portion and a distal portion. The scanner assembly 110 is positioned at a distal portion of the flexible elongate member 115. The flexible elongate member 115 includes a longitudinal axis LA. The longitudinal axis LA may be associated with the IVUS device 102 and/or the imaging assembly 110.

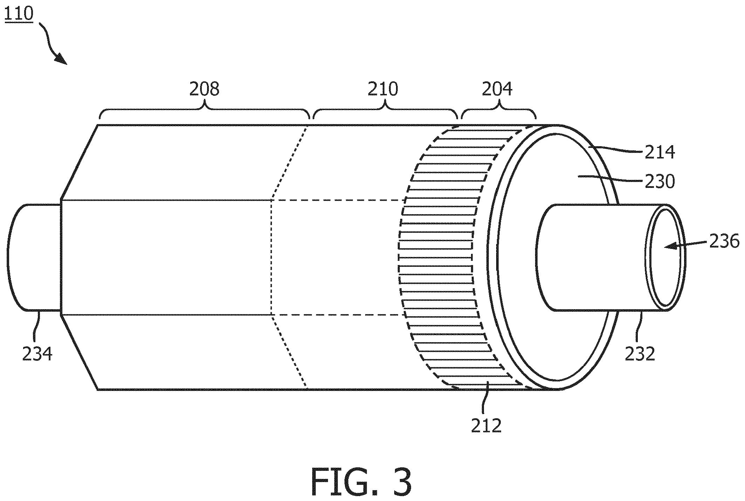

Fig. 2 is a top view of a portion of an ultrasound scanner assembly 110 according to an embodiment of the present disclosure. The assembly 110 includes a transducer array 124 formed in a transducer region 204 and transducer control logic dies 206 (including dies 206A and 206B) formed in a control region 208, with a transition region 210 disposed therebetween. The transducer control logic dies 206 and the transducers 212 are mounted on a flex circuit 214 that is shown in a flat configuration in Fig. 2. Fig. 3 illustrates a rolled configuration of the flex circuit 214. The transducer array 202 is a non-limiting example of a medical sensor element and/or a medical sensor element array. The transducer control logic dies 206 is a non-limiting example of a control circuit. The transducer region 204 is disposed adjacent a distal portion 221 of the flex circuit 214. The control region 208 is disposed adjacent the proximal portion 222 of the flex circuit 214. The transition region 210 is disposed between the control region 208 and the transducer region 204. Dimensions of the transducer region 204, the control region 208, and the transition region 210 (e.g., lengths 225, 227, 229) can vary in different embodiments. In some embodiments, the lengths 225, 227, 229 can be substantially similar or a length 227 of the transition region 210 can be greater than lengths 225, 229 of the transducer region and controller region, respectively. While the imaging assembly 110 is described as including a flex circuit, it is understood that the transducers and/or controllers may be arranged to form the imaging assembly 110 in other configurations, including those omitting a flex circuit.

The transducer array 124 may include any number and type of ultrasound transducers 212, although for clarity only a limited number of ultrasound transducers are illustrated in Fig. 2. In an embodiment, the transducer array 124 includes 64 individual ultrasound transducers 212. In a further embodiment, the transducer array 124 includes 32 ultrasound transducers 212. Other numbers are both contemplated and provided for. With respect to the types of transducers, in an embodiment, the ultrasound transducers 212 are piezoelectric micro machined ultrasound transducers (PMUTs) fabricated on a micro electromechanical system (MEMS) substrate using a polymer piezoelectric material, for example as disclosed in U.S. Patent 6,641,540. In alternate embodiments, the transducer array includes piezoelectric zirconate transducers (PZT) transducers such as bulk PZT transducers, capacitive micromachined ultrasound transducers (cMUTs), single crystal piezoelectric materials, other suitable ultrasound transmitters and receivers, and/or combinations thereof.

The scanner assembly 110 may include various transducer control logic, which in the illustrated embodiment is divided into discrete control logic dies 206. In various examples, the control logic of the scanner assembly 110 performs: decoding control signals sent by the PIM 104 across the cable 112, driving one or more transducers 212 to emit an ultrasonic signal, selecting one or more transducers 212 to receive a reflected echo of the ultrasonic signal, amplifying a signal representing the received echo, and/or transmitting the signal to the PIM across the cable 112. In the illustrated embodiment, a scanner assembly 110 having 64 ultrasound transducers 212 divides the control logic across nine control logic dies 206, of which five are shown in Fig. 2. Designs incorporating other numbers of control logic dies 206 including 8, 9, 16, 17 and more are utilized in other embodiments. In general, the control logic dies 206 are characterized by the number of transducers they are capable of driving, and exemplary control logic dies 206 drive 4, 8, and/or 16 transducers.

The control logic dies are not necessarily homogenous. In some embodiments, a single controller is designated a master control logic die 206A and contains the communication interface for the cable 112. Accordingly, the master control circuit may include control logic that decodes control signals received over the cable 112, transmits control responses over the cable 112, amplifies echo signals, and/or transmits the echo signals over the cable 112. The remaining controllers are slave controllers 206B. The slave controllers 206B may include control logic that drives a transducer 212 to emit an ultrasonic signal and selects a transducer 212 to receive an echo. In the depicted embodiment, the master controller 206A does not directly control any transducers 212. In other embodiments, the master controller 206A drives the same number of transducers 212 as the slave controllers 206B or drives a reduced set of transducers 212 as compared to the slave controllers 206B. In an exemplary embodiment, a single master controller 206A and eight slave controllers 206B are provided with eight transducers assigned to each slave controller 206B.

The flex circuit 214, on which the transducer control logic dies 206 and the transducers 212 are mounted, provides structural support and interconnects for electrical coupling. The flex circuit 214 may be constructed to include a film layer of a flexible polyimide material such as KAPTON™ (trademark of DuPont). Other suitable materials include polyester films, polyimide films, polyethylene napthalate films, or polyetherimide films, other flexible printed semiconductor substrates as well as products such as Upilex® (registered trademark of Ube Industries) and TEFLON® (registered trademark of E.I. du Pont). In the flat configuration illustrated in Fig. 2, the flex circuit 214 has a generally rectangular shape. As shown and described herein, the flex circuit 214 is configured to be wrapped around a support member 230 (Fig. 3) to form a cylindrical toroid in some instances. Therefore, the thickness of the film layer of the flex circuit 214 is generally related to the degree of curvature in the final assembled scanner assembly 110. In some embodiments, the film layer is between 5 µm and 100 µm, with some particular embodiments being between 12.7 µm and 25.1 µm.

To electrically interconnect the control logic dies 206 and the transducers 212, in an embodiment, the flex circuit 214 further includes conductive traces 216 formed on the film layer that carry signals between the control logic dies 206 and the transducers 212. In particular, the conductive traces 216 providing communication between the control logic dies 206 and the transducers 212 extend along the flex circuit 214 within the transition region 210. In some instances, the conductive traces 216 can also facilitate electrical communication between the master controller 206A and the slave controllers 206B. The conductive traces 216 can also provide a set of conductive pads that contact the conductors 218 of cable 112 when the conductors 218 of the cable 112 are mechanically and electrically coupled to the flex circuit 214. Suitable materials for the conductive traces 216 include copper, gold, aluminum, silver, tantalum, nickel, and tin, and may be deposited on the flex circuit 214 by processes such as sputtering, plating, and etching. In an embodiment, the flex circuit 214 includes a chromium adhesion layer. The width and thickness of the conductive traces 216 are selected to provide proper conductivity and resilience when the flex circuit 214 is rolled. In that regard, an exemplary range for the thickness of a conductive trace 216 and/or conductive pad is between 10-50 µm. For example, in an embodiment, 20 µm conductive traces 216 are separated by 20 µm of space. The width of a conductive trace 216 on the flex circuit 214 may be further determined by the width of the conductor 218 to be coupled to the trace/pad.

The flex circuit 214 can include a conductor interface 220 in some embodiments. The conductor interface 220 can be a location of the flex circuit 214 where the conductors 218 of the cable 112 are coupled to the flex circuit 214. For example, the bare conductors of the cable 112 are electrically coupled to the flex circuit 214 at the conductor interface 220. The conductor interface 220 can be tab extending from the main body of flex circuit 214. In that regard, the main body of the flex circuit 214 can refer collectively to the transducer region 204, controller region 208, and the transition region 210. In the illustrated embodiment, the conductor interface 220 extends from the proximal portion 222 of the flex circuit 214. In other embodiments, the conductor interface 220 is positioned at other parts of the flex circuit 214, such as the distal portion 221, or the flex circuit 214 omits the conductor interface 220. A value of a dimension of the tab or conductor interface 220, such as a width 224, can be less than the value of a dimension of the main body of the flex circuit 214, such as a width 226. In some embodiments, the substrate forming the conductor interface 220 is made of the same material(s) and/or is similarly flexible as the flex circuit 214. In other embodiments, the conductor interface 220 is made of different materials and/or is comparatively more rigid than the flex circuit 214. For example, the conductor interface 220 can be made of a plastic, thermoplastic, polymer, hard polymer, etc., including polyoxymethylene (e.g., DELRIN®), polyether ether ketone (PEEK), nylon, and/or other suitable materials. As described in greater detail herein, the support member 230, the flex circuit 214, the conductor interface 220 and/or the conductor(s) 218 can be variously configured to facilitate efficient manufacturing and operation of the scanner assembly 110.

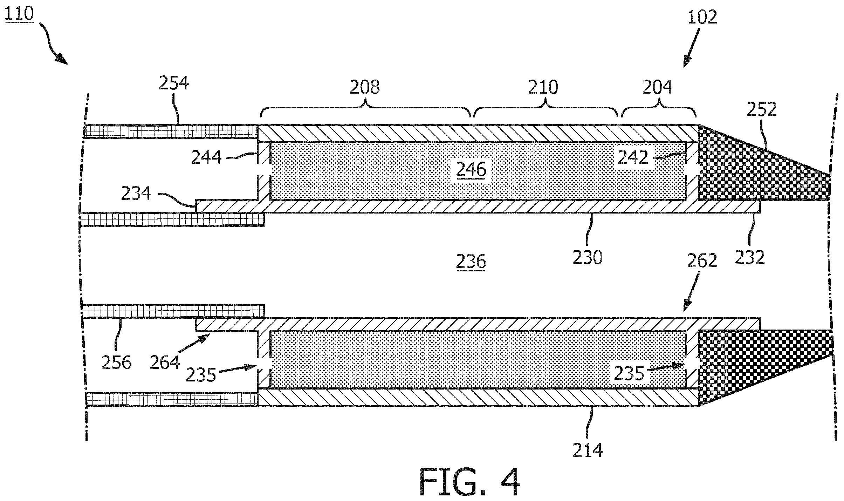

In some instances, the scanner assembly 110 is transitioned from a flat configuration (Fig. 2) to a rolled or more cylindrical configuration (Figs. 3 and 4). For example, in some embodiments, techniques are utilized as disclosed in one or more of U.S. Patent No. 6,776,763, titled "ULTRASONIC TRANSDUCER ARRAY AND METHOD OF MANUFACTURING THE SAME" and U.S. Patent No. 7,226,417, titled "HIGH RESOLUTION INTRAVASCULAR ULTRASOUND TRANSDUCER ASSEMBLY HAVING A FLEXIBLE SUBSTRATE".

As shown in Figs. 3 and 4, the flex circuit 214 is positioned around the support member 230 in the rolled configuration. Fig. 3 is a diagrammatic side view with the flex circuit 214 in the rolled configuration around the support member 230, according to aspects of the present disclosure. Fig. 4 is a diagrammatic cross-sectional side view of a distal portion of the IVUS device 102, including the flex circuit 214 and the support member 230, according to aspects of the present disclosure.

The support member 230 can be referenced as a unibody in some instances. The support member 230 can be composed of a metallic material, such as stainless steel, or non-metallic material, such as a plastic or polymer as described in U.S. Provisional Application No. 61/985,220, "Pre-Doped Solid Substrate for Intravascular Devices," filed April 28, 2014. The support member 230 can be ferrule having a distal portion 262 and a proximal portion 264. The support member 230 can define a lumen 236 extending longitudinally therethrough. The lumen 236 is in communication with the exit port 116 and is sized and shaped to receive the guide wire 118 (Fig. 1). The support member 230 can be manufactured accordingly to any suitable process. For example, the support member 230 can be machined, such as by removing material from a blank to shape the support member 230, or molded, such as by an injection molding process. In some embodiments, the support member 230 may be integrally formed as a unitary structure, while in other embodiments the support member 230 may be formed of different components, such as a ferrule and stands 242, 244, that are fixedly coupled to one another.

Stands 242, 244 that extend vertically are provided at the distal and proximal portions 262, 264, respectively, of the support member 230. The stands 242, 244 elevate and support the distal and proximal portions of the flex circuit 214. In that regard, portions of the flex circuit 214, such as the transducer portion 204, can be spaced from a central body portion of the support member 230 extending between the stands 242, 244. The stands 242, 244 can have the same outer diameter or different outer diameters. For example, the distal stand 242 can have a larger or smaller outer diameter than the proximal stand 244. To improve acoustic performance, any cavities between the flex circuit 214 and the surface of the support member 230 are filled with a backing material 246. The liquid backing material 246 can be introduced between the flex circuit 214 and the support member 230 via passageways 235 in the stands 242, 244. In some embodiments, suction can be applied via the passageways 235 of one of the stands 242, 244, while the liquid backing material 246 is fed between the flex circuit 214 and the support member 230 via the passageways 235 of the other of the stands 242, 244. The backing material can be cured to allow it to solidify and set. In various embodiments, the support member 230 includes more than two stands 242, 244, only one of the stands 242, 244, or neither of the stands. In that regard the support member 230 can have an increased diameter distal portion 262 and/or increased diameter proximal portion 264 that is sized and shaped to elevate and support the distal and/or proximal portions of the flex circuit 214.

The support member 230 can be substantially cylindrical in some embodiments. Other shapes of the support member 230 are also contemplated including geometrical, non-geometrical, symmetrical, non-symmetrical, cross-sectional profiles. Different portions the support member 230 can be variously shaped in other embodiments. For example, the proximal portion 264 can have a larger outer diameter than the outer diameters of the distal portion 262 or a central portion extending between the distal and proximal portions 262, 264. In some embodiments, an inner diameter of the support member 230 (e.g., the diameter of the lumen 236) can correspondingly increase or decrease as the outer diameter changes. In other embodiments, the inner diameter of the support member 230 remains the same despite variations in the outer diameter.

A proximal inner member 256 and a proximal outer member 254 are coupled to the proximal portion 264 of the support member 230. The proximal inner member 256 and/or the proximal outer member 254 can be flexible elongate member that extend from proximal portion of the IVUS device 102, such as the proximal connector 114, to the imaging assembly 110. For example, the proximal inner member 256 can be received within a proximal flange 234. The proximal outer member 254 abuts and is in contact with the flex circuit 214. A distal member 252 is coupled to the distal portion 262 of the support member 230. The distal member 252 can be a flexible component that defines a distal most portion of the IVUS device 102. For example, the distal member 252 is positioned around the distal flange 232. The distal member 252 can abut and be in contact with the flex circuit 214 and the stand 242. The distal member 252 can be the distal-most component of the IVUS device 102.

One or more adhesives can be disposed between various components at the distal portion of the IVUS device 102. For example, one or more of the flex circuit 214, the support member 230, the distal member 252, the proximal inner member 256, and/or the proximal outer member 254 can be coupled to one another via an adhesive.

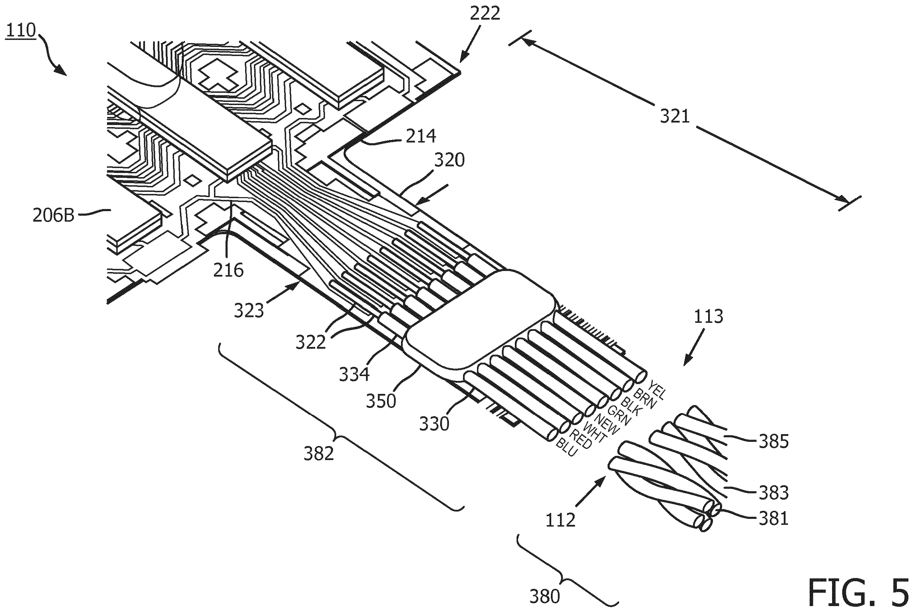

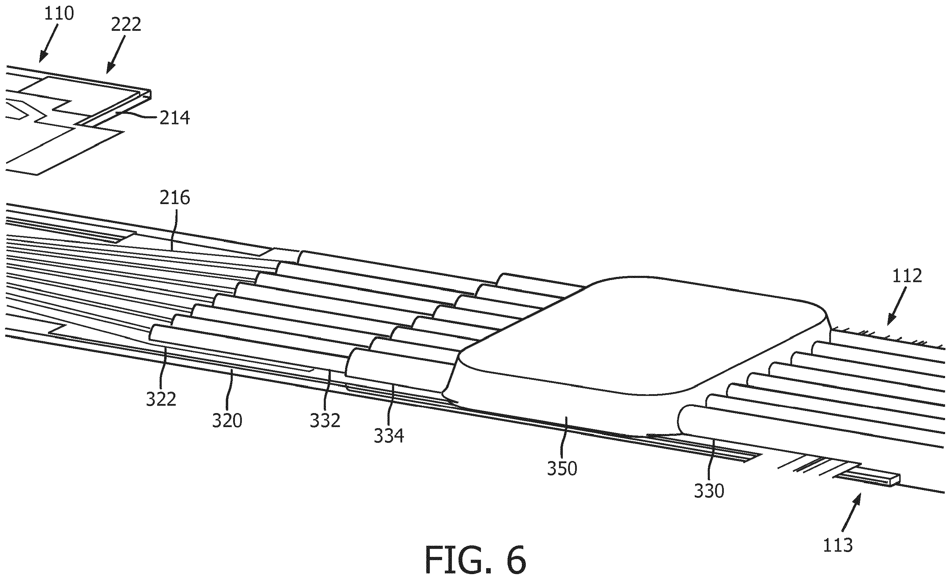

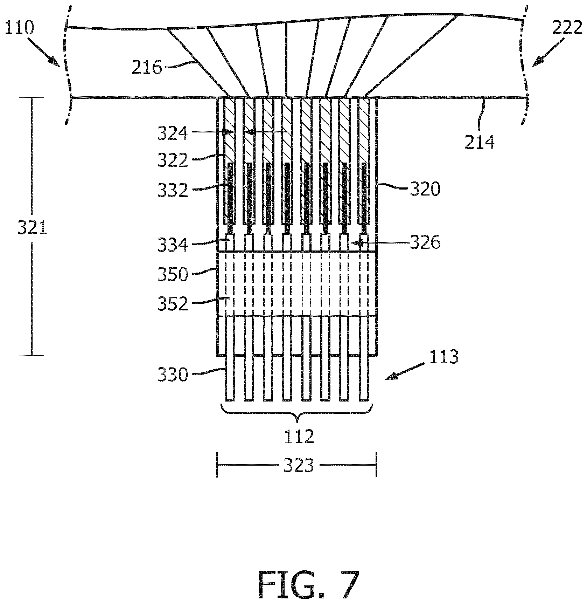

Figs. 5, 6, and 7 illustrate an exemplary embodiment of a flex circuit 214 coupled to an electrical cable 112. Figs. 5 and 6 show different perspective views, and Fig. 7 shows a top view. Wires 330 of the electrical cable 112 are coupled to the flex circuit 214 at a conductor interface 320. For example, each of the wires 330 is soldered or welded to a corresponding conductive pad 322 of the conductor interface 320. The wires 330 extend through a guide member 350. The guide member 350 is positioned adjacent to the conductor interface 320. The guide member 350 laterally and/or longitudinally aligns the wires 330 with respective conductive pads 322 of the conductor interface 320. Thus, advantageously, the intraluminal device may be more efficiently assembled by aligning the guide member 350 to the conductor interface 320, rather than individually aligning each wire 330 with the corresponding conductive pad 322. The guide member 350 can be mechanically coupled to the conductor interface 320, such as by an adhesive. Thus, advantageously, the wires 330 are more securely connected to the flex circuit 214 to form a more robust imaging assembly 110, compared to an imaging assembly in which only solder is used is mechanically coupled to the wires 330 to the flex circuit 214.

The conductor interface 320 extends proximally from the proximal portion 222 of the flex circuit 214. The features of the conductor interface 320 can be similar to those of the conductor interface 220 (Fig. 2). A length 321 and a width 323 of the conductor interface 320 can be any suitable value. In some embodiments, the flex circuit 214 can have multiple conductor interfaces 320, including two, three, four or more. One or more of the conductor interfaces 320 can extend from the distal portion 221, the proximal portion 222, the side portions, and/or any suitable location of the flex circuit 214.

The conductor interface 320 includes the conductive pads 322. The conductive pads 322 are directly or indirectly in electrical communication with one or more components of the imaging assembly 110 such as the controllers 206A, 206B and/or the transducers 212 (Fig. 2). For example, the conductive traces 216 can facilitate communication between conductive pads 322, the controllers 206A, 206B, and/or the transducers 212. In some embodiments, the conductive pads 322 and the conductive traces 216 are similarly sized and shaped and/or formed of similar conductive materials. In some embodiments, the conductive traces 216 extend within the flex circuit 214, and the conductive pads 322 are disposed on a surface of the flex circuit 214. In some embodiments, the conductive pads 322 are sized and shaped to facilitate coupling to the conductors 332 of the wires 330 forming the electrical cable 112. For example, the conductive pads 322 can be relatively wider than the conductive traces 216. Accordingly, the conductors 332 can be more easily positioned on and soldered and/or welded to the conductive pads 322 to establish electrical communication between the cable 112, the controllers 206A, 206B, and/or the transducers 212. In that regard, the individual conductive pads 322 can be spaced from one another, in a direction perpendicular to the longitudinal axis LA of the imaging assembly 110, by any suitable distance 324. In some embodiments, the spacing of the conductive pads 322 is based on the voltages carried by the conductors 332. For example, the high voltage conductors 332 and/or conductive pads 322 can be spaced relatively farther away from the low voltage conductors and/or conductive pads.

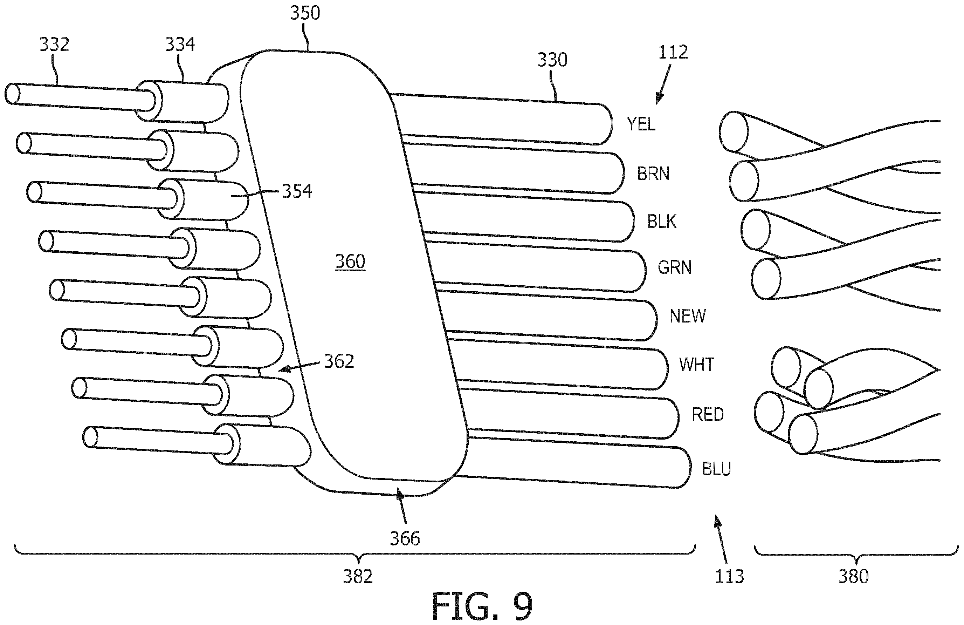

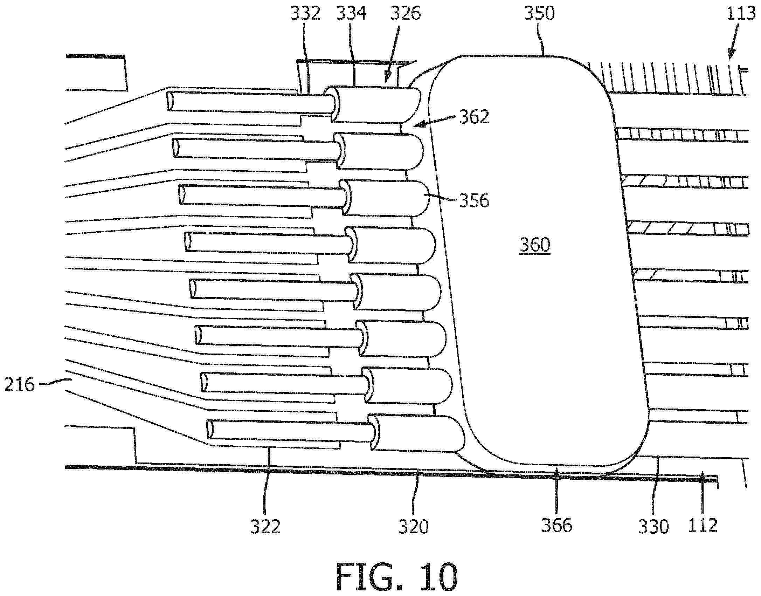

A distal portion 113 of electrical cable 112 is shown in Figs. 5, 6, 7, 9, and 10. Fig. 9 is a perspective view of the electrical cable 112 and the guide member 350. Fig. 10 is a perspective view of the electrical cable 112 coupled to the scanner assembly 110 at the conductor interface 320. In the illustrated embodiment, the electrical cable 112 includes eight wires 330. It is understood that in other embodiments, the electrical cable 112 may have any suitable number of wires 330, including two, three, four, or more. Each wire 330 can have any suitable structure. For example, the wires 330 can include a bare conductor 332 surrounded by an insulation layer 334, as shown. In some embodiments, individual wires 330 and/or groupings of wires 330 can be further surrounded by additional insulation layer(s) or jacket(s). The wires 330 of the electrical cable 112 extend along a length of the IVUS device 102 (Fig. 1), such as from the connector 114 at the proximal portion to the imaging assembly 110 at the distal portion.

Referring to Figs. 5 and 9, at a distal-most segment 382 of the electrical cable 112, the wires 330 extend linearly and are positioned side-by-side, adjacent to one another. At a more proximal segment 380, the wires 330 are twisted together. For example, different subsets of the wires 330 can be twisted together in clockwise and/or counterclockwise directions. In the illustrated embodiment, four of the wires 330 are twisted together in a subset 381, two of the wires 330 are twisted together in a subset 383, and another two of the wires 330 are twisted together in a subset 385. The wires 330 transition from the twisted configuration to the linear configuration at the distal portion 113 of the electrical cable 112.

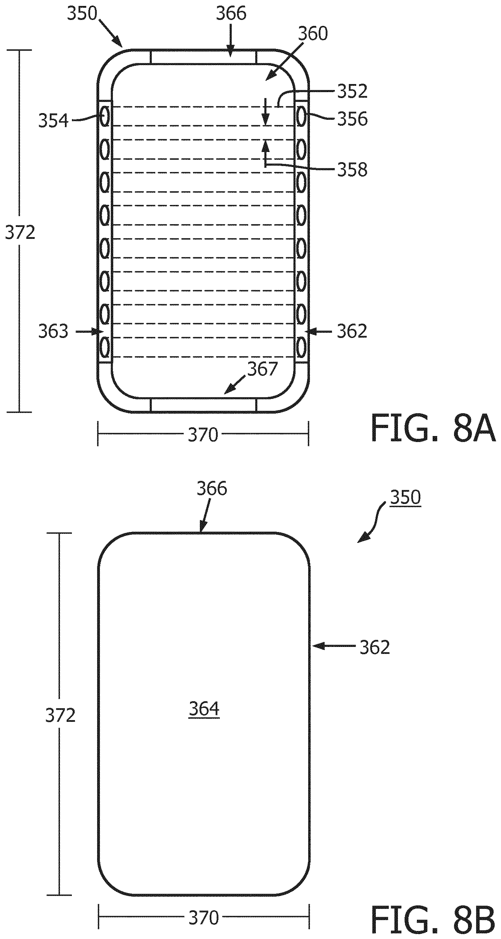

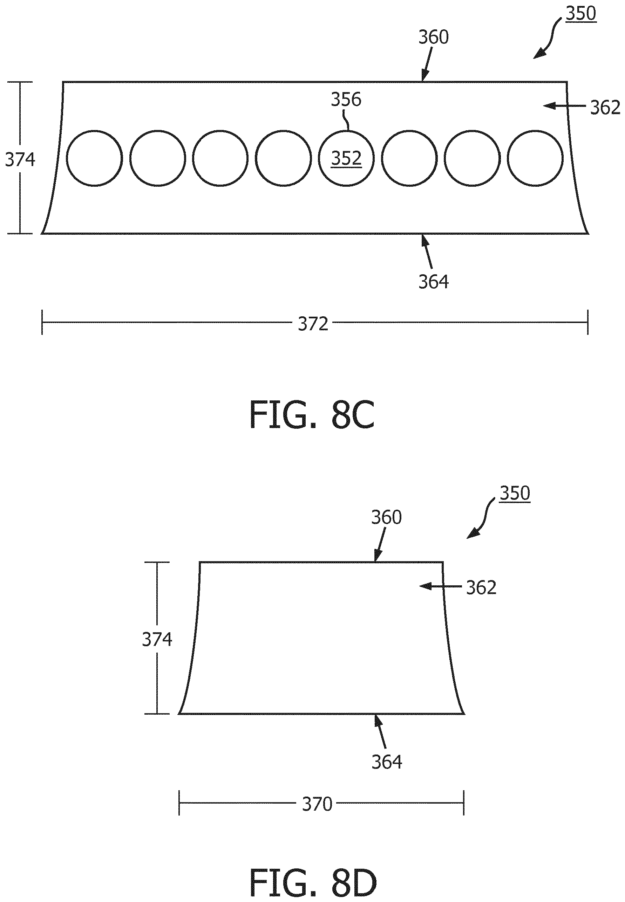

Referring to Figs. 5, 6, 7, 9, and 10, the wires 330 at the distal portion 113 of the electrical cable 112 extend longitudinally through the guide member 350. The guide member 350 is also illustrated in Figs. 8A, 8B, 8C, and 8D. Fig. 8A is a top view of the guide member 350 showing a top surface 360. Fig. 8B is a bottom view of the guide member 350 showing a bottom surface 364. Fig. 8C is a proximal view of the guide member 350 showing a proximal surface 362 with proximal openings 356 for bores or lumens 352. Fig. 8D is a side view of the guide member showing a side surface 366.

In the illustrated embodiment, individual wires 330 extend through respective bores 352 of the guide member 350. In such embodiments, the number of bores 352 equals the number of wires 330. For example, the guide member 350 can include eight bores 352. In other embodiments, two or more wires can extend through a single bore. In such embodiments, the number of bores is less than the number of wires. The bores 352 extend longitudinally through the guide member 350, between a distal opening 354 and a proximal opening 356. The illustrated embodiment shows the bore 352, the distal opening 354, and the proximal opening 356 are circularly shaped. In other embodiments, the bore 352, the distal opening 354, and/or the proximal opening 356 can have any suitable shape, including a polygon, an ellipse, etc. Generally, the bore 352, the distal opening 354, and/or the proximal opening 356 can be sized and shaped to accommodate one or more wires 330. For example, a radius of the bore 352, the distal opening 354, and/or the proximal opening 356 may be selected based on a radius of the wire 330. For example, the radius of the bore 352, the distal opening 354, and/or the proximal opening 356 may be greater than or equal to the radius of the wire 330.

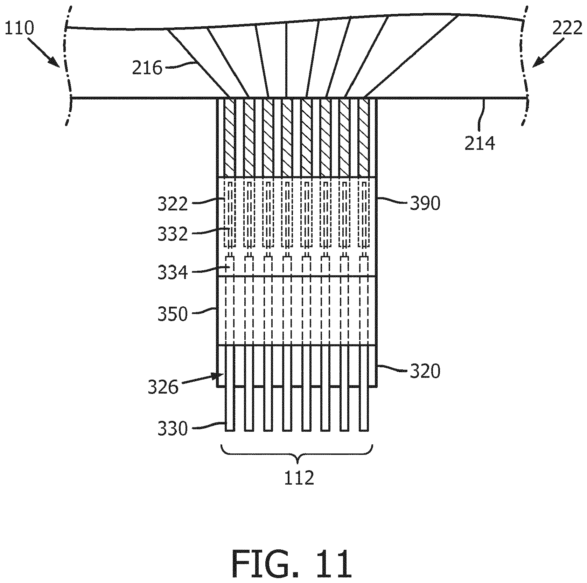

The guide member 350 maintains the linear configuration of the wires 330 by positioning the wires within the bores 352 at a fixed distance 358 from one another. That is, the lateral positions of the wires 330 may be fixed by positioning the wires within the bores 352. As shown in Fig. 8A, for example, the individual bores 352 can be spaced from one another, in a direction perpendicular to the longitudinal axis LA of the imaging assembly 110, by any suitable distance 358. In some instances, the spacing can be based on the size of the conductors 332 and/or the wires 330. The distance 358 between the bores 352 and the distance 324 between the conductive pads 322 (Fig. 7) can be related such that the conductors 332 are laterally aligned with the conductive pads 322 when the guide member 350 is disposed adjacent to the conductor interface 320. In various embodiments, the distance 324 and the distance 358 are the same or different. In either case, the distances 324, 358 are selected such that the conductors 332 are laterally aligned with the conductive pads 322 when the guide member 350 is laterally aligned with the conductor interface 320, as shown in Figs. 5, 6, 7, and 10. The guide member 350 can be longitudinally positioned along the conductor interface 320 such that the bare conductors 332 are longitudinally aligned with the conductive pads 322, as also shown in Figs. 5, 6, 7, and 10. Electrical communication between the electrical cable 112 and the components of the flex circuit 214 can be established by soldering, welding, and/or otherwise electrically coupling the conductors 332 and the conductive pads 322. In some embodiments, such as those described with respect to Figs. 11-13 herein, electrical communication is alternatively established.

Referring again to Figs. 8A, 8B, 8C, and 8D, the guide member 350 can have any suitable dimensions 370, 372, 374. The length 370, the width 372, and/or the height 374 of the guide member 350 can be any suitable value(s). In some embodiments, the guide member 350 can be adhesive that is formed around the conductors 332. For example, one or more the dimensions 370, 372, 374, and/or as well as the distance 358 can be defined by the dimensions of the conductors themselves. In some instances, the height 374 can be selected to be as thin as possible while still maintaining ability to align the wires 330. For example, the minimum height 374 can be limited by the thickness of wires 330.

Referring to Fig. 7, 8A, 8B, and 8C, one or dimensions of the guide member 350 can be based on one or more dimensions of the conductor interface 320. For example, the width 372 of the guide member 350 can be equal to the width 323 of the conductor interface 320. In such embodiments, the guide member 350 is laterally aligned to the conductor interface 320 by aligning the widths 323, 372. The guide member 350 is longitudinally positioned along the conductor interface 320 so that the bare conductors 332 are positioned adjacent to the conductive pads 322. Because the guide member 350 maintains the spacing between the wires 330 such that the wires 330 are aligned with the conductive pads 322, the wires 330 can be more easily aligned with the conductive pads 322 by aligning the widths 323, 372 of the guide member 350 and the conductor interface 320, compared to individually positioning the wires 330 on the respective conductive pad 322. That is, rather than individually aligning, both longitudinally and laterally, each of the wires 330 with a corresponding conductive pad 322, all of the wires 330 can be collectively aligned, both longitudinally and laterally, with respective conductive pads 322 by aligning the guide member 350 and the conductor interface 320.

Referring to Figs. 8A, 8B, 8C, and 8D, generally, the guide member 350 may have any suitable shape, including a rectangular prism, polyhedron, ellipsoid, prism, pyramid, etc. The surfaces 360, 362, 363, 364, 366, 367 of the guide member 350 may be planar and/or curved. For example, in the illustrated embodiment, the top surface 360 and the bottom surface 364 are planar and generally rectangular-shaped. A distal surface 363, the proximal surface 362, a side surface 367, and the side surface 366 are curved. In some embodiments, the surface areas of the top surface 360 and the bottom surface 364 are equal. In other embodiments, such as the illustrated embodiment, the surface areas of the top surface 360 and the bottom surface 364 are different. For example, the surface area of the bottom surface 364 is greater than a surface area of the top surface 360. In that regard, the length and/or width of the top surface 360 may be less than the length and/or width of the bottom surface 364.

Referring to Figs. 5, 6, 7, and 10, the guide member 350 is positioned adjacent to the conductor interface 320. For example, the guide member 350 can be disposed adjacent to a top surface 326 of the conductor interface 320. In some embodiments, the guide member 350 is coupled to the conductor interface 320. For example, the bottom surface 364 of the guide member 350 can be affixed to the top surface 326 of the conductor interface 320, such as by an adhesive. Any suitable adhesive may be utilized, including epoxies with and without fillers or solder, for example. In some embodiments, the adhesive can be applied to top surface 326 and/or the bottom surface 364 prior to the surfaces 326, 364 being moved into contact with another. Attachment of the guide member 350 and the conductor interface 320 establishes a robust mechanical interface between the electrical cable 112 and the flex circuit 214. In that regard, the entire surface area of the bottom surface 364 of the guide member 350 (Fig. 8B) is utilized to establish a mechanical interface, rather than only the soldered/welded portions of the conductor 332 and the conductive pads 322. Further, an adhesive that is configured to withstand forces, such as pulling, is used to join the conductor interface 320 and the guide member 350. The surfaces 326, 364 are joined together with the guide member 350 and the conductor interface 320 aligned. When the guide member 350 is coupled to the conductor interface 320, at least a distal portion of the wires 330 is fixed, laterally and/or longitudinally, relative to the flex circuit 214.

The guide member 350 can be composed of a metallic material, such as stainless steel, or non-metallic material, such as a plastic or polymer. In some embodiments, the guide member 350 is formed separately from the wires 330. For example, any suitable manufacturing process, such as a machining, injection molding, three-dimensional printing, etc., may be utilized to manufacture the guide member 350. In such embodiments, each of the wires 330 is threaded or translated longitudinally through a corresponding bore 352 such that the wires 330 extend longitudinally through the guide member 350. In some embodiments, the guide member 350 is formed around the wires 330. For example, the guide member 350 may be molded around the wires 330. In such embodiments, the guide member 350 is formed and the wires 330 are positioned to extend through the guide member 350 in a single step.

The wires 330 of the electrical cable 112 may be positioned within the bores 352 of the guide member 350 such that the wires do not move longitudinally. For example, an adhesive may be used to fix the longitudinal position of the wires 330 relative to the guide member 350. In some embodiments, the distal ends of the wires 330 can be longitudinally aligned after the wires 330 are positioned within the bores 352. For example, the distal ends of the conductors 332 may be aligned after the wires 330 are thread through the bores 352. In some instances, the distal ends of the wires 330 are aligned before the wires 330 are positioned within the bores 352. For example, the distal ends of the conductors 332 can be aligned and then the guide member 350 can be formed around the wires 330.

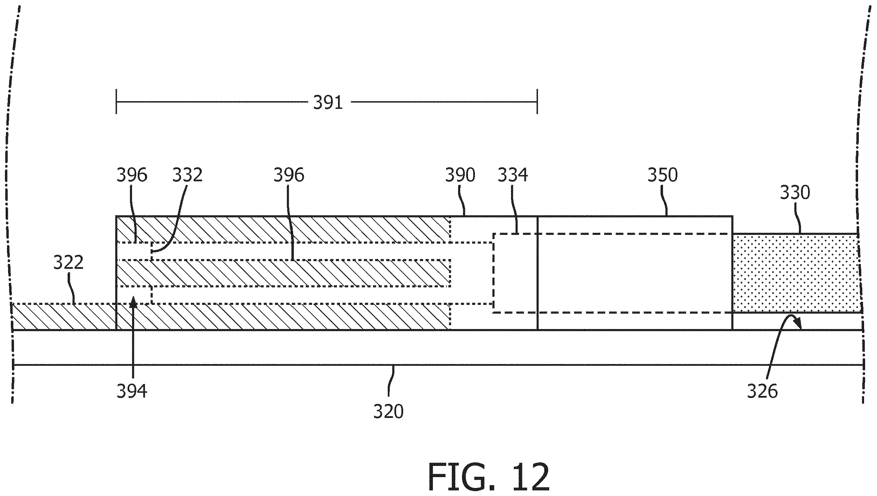

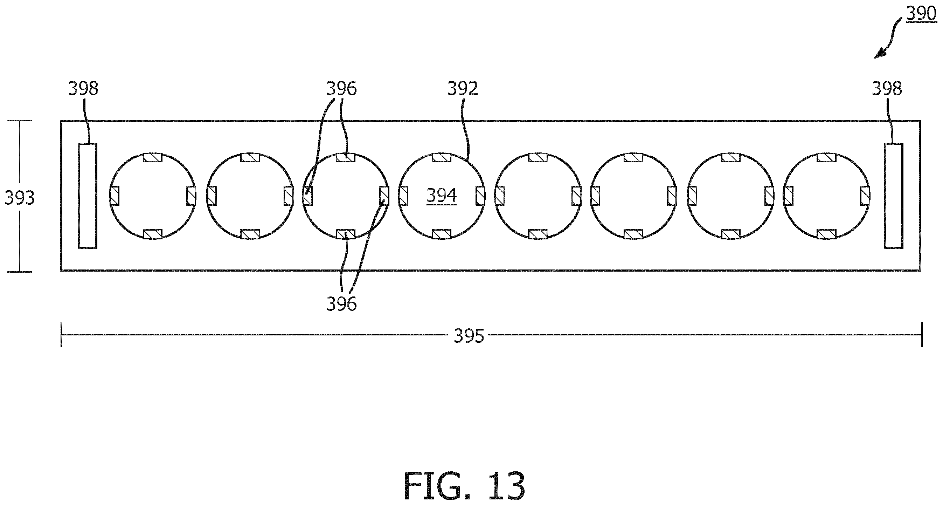

Fig. 11, Fig. 12, and Fig. 13 illustrate an embodiment of the imaging assembly 110 including a guide member 390 and the guide member 350. The guide member 390 and the guide member 350 together facilitate coupling the electrical cable 112 and the flex circuit 214. Fig. 11 is a top view and Fig. 12 is side view of a proximal portion of the imaging assembly 110, including the guide member 350, the guide member 390, and the wires 330 of the electrical cable. Fig. 13 is a proximal view of the guide member 390. The guide member 390 includes conductive members 396 that contact the bare conductors 332 of the wires 330. In that regard, electrical communication between the flex circuit 214 and the electrical cable 112 can be established via the conductive members 396, rather than by soldering/welding conductors 332 at the conductor interface 320. The guide member 390 may be coupled to the conductor interface 320, such as by an adhesive. During assembly, the guide member 350, with the wires 330 extending therethrough, can be mechanically attached to the guide member 390 to align the wires 330 to respective conductive portions 322 and/or 396, as well as to mechanically couple the electrical cable and the flex circuit 214.

The guide member 390 can have features similar to the guide member 350. For example, the guide member 390 may have any suitable shape, including a rectangular prism, polyhedron, ellipsoid, prism, pyramid, etc. The guide member 390 can be composed of a metallic material, such as stainless steel, or non-metallic material, such as a plastic or polymer. Referring to Figs. 12 and 13, the length 391 and/or width 395 of the guide member 390 can be any suitable value.

Referring to Fig. 13, the wires 330 are inserted into the bores 394 through the proximal opening 392. The bores 394 are sized and shaped to accommodate the bare conductors 332 and/or the insulation layer 334 of the wires 330. A radius of the bore 394 and/or the proximal opening 392 may be selected based on a radius of the wire 330. For example, the radius of the bore 394 and/or the proximal opening 392 may be greater than or equal to the radius of the wire 330.

The guide member 390 can include one, two, three, four, or more conductive segments 322, 396. The conductive segments 322, 396 are in electrical communication with the controllers 206A, 206B and the transducers 212 of the flex circuit 214. Generally, the conductive segments 322, 396 can be sized, shaped, and positioned within the guide member 390 to allow the bare conductors 332 to contact the conductive segments 322, 396 when the wires 330 are inserted into the guide member 390. Exemplary configurations of the conductive segments 322, 396 are illustrated in Figs. 11-13. In that regard, Fig. 11 shows an embodiment in which at least a portion of the conductive pad 322 extends longitudinally within the guide member 390. The bare conductor 332 contacts the conductive pad 322. A portion of the conductive pad 322 and conductive members 396 extend longitudinally within the guide member 390 of Fig. 12. The bare conductor 332 contacts the conductive pads 322 and/or the conductive members 396. Fig. 13 illustrates an embodiment of the guide member 390 include conductive members 396 positioned circumferentially around the lumen or bore 394. When the wires 330 are inserted into the guide member 39, the conductive members 396 are configured to contact the bare conductor 332. In some embodiments, the conductor interface 320 omits the conductive pads 322. For example, the conductive segments 396 can be in electrical communication with the controllers 206A, 206B and the transducers 212 of the flex circuit 214 via the conductive traces 216.

The guide member 390 is coupled to the conductor interface 320. For example, the bottom surface of the guide member 390 can be affixed to the top surface 326 of the conductor interface 320, such as by an adhesive. In some embodiments, the flex circuit 214 can be manufactured with the guide member 390 coupled to the conductor interface 320.

The guide members 350, 390 can include an engagement mechanism to couple the two components. For example, as shown in Fig. 13, the guide member 390 includes an attachment member 398. The guide member 350 can include a corresponding attachment member that engages the attachment member 398. During assembly of the IVUS device 102, the guide member 350, with the wires 330 extending therethrough, is brought into contact with the guide member 390. The wires 330 are inserted into the bores 394 of the guide member 390 and the attachment member 398 of the guide member 390 engages the corresponding attachment member of the guide member 350. In some embodiments, only the guide member 390, and not the guide member 350, is coupled to the conductor interface 320. In some embodiments, both guide members 350, 390 are coupled to the conductor interface 320. For example, the guide member 350 can be coupled to the guide member 350 substantially simultaneously or after the guide member 350 is coupled to the guide member 390.

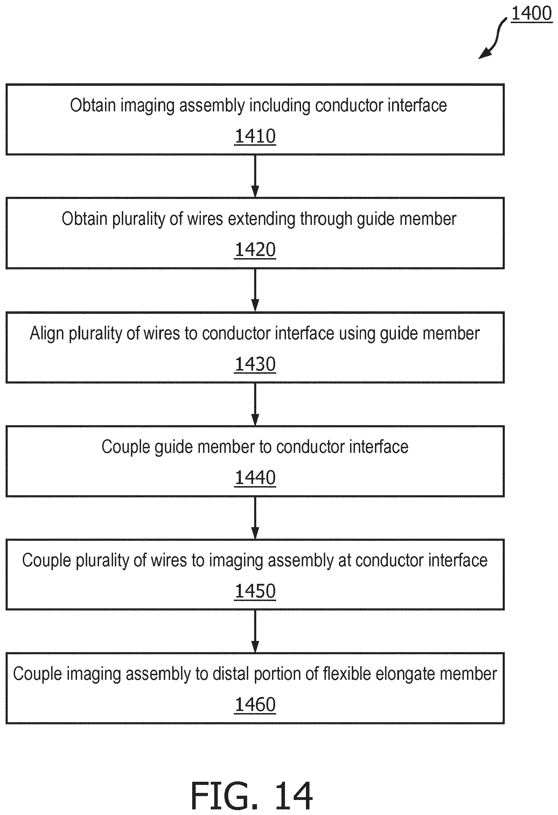

Fig. 14 is a flow diagram of a method 1400 of assembling an intraluminal imaging device, as described herein. It is understood that the steps of method 1400 may be performed in a different order than shown in Fig. 14, additional steps can be provided before, during, and after the steps, and/or some of the steps described can be replaced or eliminated in other embodiments. The steps of the method 1400 can be carried out by a manufacturer of the intraluminal imaging device.

At step 1410, the method 1400 includes obtaining an imaging assembly including a conductor interface. For example, the imaging assembly can include an imaging assembly, such as a flex circuit having multiple transducers and controllers. The conductor interface can extend from the flex circuit, such as the proximal portion of the flex circuit. The conductor interface can include multiple conductive pads in electrical communication with the transducers and controllers of the imaging assembly.

At step 1420, the method 1400 includes obtaining a plurality of wires extending through a guide member. For example, the plurality of wires can extend through a plurality of bores of the guide member. The guide member may be disposed at a distal portion of the electrical wires. In some embodiments, the method 1400 can include forming the guide member around the wires. In some embodiments, the method 1400 can include positioning the plurality of wires through bores of the guide members. The wires can be coupled to the guide member, as a result of forming the guide member around the wires, via an adhesive, and/or mechanical engagement of the insulation layer(s) of the wire and the surface of the bores of the guide member.

At step 1430, the method 1400 includes aligning the plurality of wires with the conductor interface using the guide member. For example, widths of the conductor interface and the guide member can be aligned such that that each wire is laterally aligned with a respective conductive pad of the conductive interface. In some embodiments, the guide member longitudinally aligned with the conductor interface such that that the bare conductors of the wires are longitudinally aligned with the conductive pads.

At step 1440, the method 1400 includes coupling the guide member to the conductor interface. For example, the adhesive can be applied to the bottom surface of the guide member and/or the top surface of the conductor interface. The bottom surface of the guide member and the top surface of the conductor interface can be brought into contact to affix the components together. Step 1440 results in creating a robust mechanical interface between the wires and the flex circuit.

At step 1450, the method 1400 includes coupling the plurality of wires to imaging assembly at the conductor interface. For example, step 1450 can include soldering/welding each wire to a respective conductive pad of the conductor interface. In some embodiments, step 1450 establishes electrical communication without soldering/welding. For example, a further guide member can be coupled to the conductor interface. The further guide member includes a plurality of bores. Step 1450 can include inserting each of the plurality of wires into a respective bore of the plurality of bores of the further guide member. The bores of the further guide member can include conductive portions that contact the bare conductors. Accordingly, electrical communication between the wires and the imaging assembly is established when the conductors of the wires contact the conductive portions of the further guide member. In some embodiments, the method 1400 includes coupling the guide member and the further guide member. For example, the guide member and further guide member can include an attachment mechanism that joins the two components when they are brought into contact with one another. In various embodiments, step 1440 can be performed before, after, and/or simultaneously as step 1450.

At step 1460, the method 1400 includes coupling the imaging assembly to a distal portion of a flexible elongate member. For example, step 1460 can include positioning the flex circuit around a support member to form an imaging assembly of the intraluminal device. The flex circuit may initially be in a flat configuration. Step 1460 can include transitioning at least a portion of the flex circuit into a rolled configuration around the support member. The flex circuit can be positioned around the support member such that the inner diameter of the flex circuit contacts a backing material disposed between the support member and the flex circuit. The method 1400 may include securing the flex circuit to the support member using one or more adhesives. The method 1400 may also include curing the backing material, such as by using heat or light. The method 1400 includes coupling the imaging assembly to one or more distal members and one or more proximal members to form the intraluminal device. In that regard, the distal member(s) and/or proximal member(s) can be coupled to the support member and/or the flex circuit. The one or more proximal members may be flexible elongate members (e.g., an inner member and/or an outer member) forming a length of the intraluminal device. The imaging assembly may be positioned at a distal portion of the intraluminal device. The distal member defines a distal-most end of the intraluminal imaging device. The method 1400 can include introducing adhesive to affix the flex circuit and the support member and/or other components of the intraluminal imaging device.

Persons skilled in the art will recognize that the apparatus, systems, and methods described above can be modified in various ways. Accordingly, persons of ordinary skill in the art will appreciate that the embodiments encompassed by the present disclosure are not limited to the particular exemplary embodiments described above. In that regard, although illustrative embodiments have been shown and described, a wide range of modification, change, and substitution is contemplated in the foregoing disclosure. It is understood that such variations may be made to the foregoing without departing from the scope of the present disclosure. Accordingly, it is appropriate that the appended claims be construed broadly and in a manner consistent with the present disclosure.

## Claims

1. An intraluminal imaging device (102), comprising:
a flexible elongate member (115) configured for positioning within a vessel of a patient, the flexible elongate member including a proximal portion and a distal portion;
an imaging assembly (110) disposed at the distal portion of the flexible elongate member, the imaging assembly including a conductor interface (320);
a plurality of wires (330) extending along a length of the flexible elongate member and in communication with the imaging assembly, wherein the plurality of wires are coupled to the imaging assembly at the conductor interface; and
a guide member (350) comprising a surface coupled to an opposing surface of the conductor interface by an adhesive, wherein the plurality of wires extend through the guide member, and wherein the guide member collectively aligns the plurality of wires to the conductor interface.

2. The device of claim 1, wherein the guide member comprises a plurality of bores (352), wherein each of the plurality of wires extends through a respective bore of the plurality of bores.

3. The device of claim 2, wherein
the conductor interface comprises a plurality of conductive pads (322), and
the guide member aligns each of the plurality of wires with a respective pad of the plurality of conductive pads.

4. The device of claim 3, wherein:
the plurality of conductive pads are spaced apart from one another by a first distance,
the plurality of bores are spaced apart from one another by a second distance, and
the first and second distances are selected such that each of the plurality of wires is aligned with the respective pad of the plurality of conductive pads.

5. The device of claim 1, wherein the guide member comprises a polymer.

6. The device of claim 1, wherein the guide member is molded around the plurality of wires.

7. The device of claim 1, wherein the plurality of wires comprise eight wires.

8. The device of claim 1, further comprising:
a further guide member (390) positioned adjacent to the conductor interface, wherein the further guide member comprises a plurality of conductive members (396) in communication with the imaging assembly, wherein the plurality of wires extend through the further guide member such that each of the plurality of wires contacts a respective conductive member of the plurality of conductive members.

9. The device of claim 8, wherein the further guide member is coupled to the guide member.

10. The device of claim 1, further comprising:
a further guide member coupled to the opposing surface of the conductor interface, wherein plurality of wires extend through the guide member and the further guide member, and wherein the guide member and the further guide member collectively align the plurality of wires to the conductor interface.

11. The device of claim 10, wherein the further guide member comprises a plurality of conductive members in communication with the imaging assembly, wherein the plurality of wires extend through the further guide member such that each of the plurality of wires contacts a respective conductive member of the plurality of conductive members.

12. The device of claim 10, wherein the guide member is coupled to the further guide member.

13. A method of assembling an intraluminal imaging device, the method comprising:
obtaining an imaging assembly including a conductor interface;
obtaining a plurality of wires extending through a guide member;
collectively aligning the plurality of wires to the conductor interface by coupling a surface of the guide member to an opposing surface of the conductor interface using an adhesive; and
coupling the plurality of wires to the imaging assembly at the conductor interface.

14. The method of claim 13, wherein the obtaining the plurality of wires extending through the guide member includes:
forming the guide member around the plurality of wires.

15. The method of claim 13, wherein the conductor interface comprises a plurality of conductive pads in communication with the imaging assembly, and wherein the coupling comprises soldering each of the plurality of wires to a respective conductive pad of the plurality of conductive pads.

## Patentansprüche

1. Ein intraluminales Bildgebungsgerät (102), das Folgendes umfasst:
ein flexibles Verlängerungselement (115) zum Positionieren innerhalb des Gefäßes eines Patienten,
wobei das flexible Verlängerungselement über einen proximalen und einem distalen Teil verfügt;
eine Bildgebungskonstruktion (110) am distalen Teil des flexiblen Verlängerungselements, wobei die Bildgebungskonstruktion eine Leiterschnittstelle (320) aufweist;
mehrere Drähte (330), die entlang der Länge des flexiblen Verlängerungselements verlaufen und mit der Bildgebungskonstruktion verbunden sind, wobei die Drähte mit an der Leiterschnittstelle mit der Bildgebungskonstruktion verbunden sind; und
ein Führungselement (350) mit einer Oberfläche, die mithilfe eines Klebstoffs mit einer gegenüberliegenden Oberfläche der Leiterschnittstelle verbunden ist, wobei die Drähte durch das Führungselement verlaufen, und wobei das Führungselement sämtliche Drähte auf die Leiterschnittstelle ausrichtet.

2. Das Gerät gemäß Anspruch 1,
wobei das Führungselement eine mehrere Bohrungen (352) umfasst, und wobei die einzelnen Drähte durch eine entsprechende Bohrung geführt werden.

3. Das Gerät gemäß Anspruch 2,
wobei die Leiterschnittstelle mehrere leitfähige Stellen (322) umfasst, und
wobei das Führungselement die einzelnen Drähte jeweils an der entsprechenden Stelle ausrichtet.

4. Das Gerät gemäß Anspruch 3, wobei:
die leitfähigen Stellen um einen ersten Abstand voneinander getrennt sind,
die Bohrungen in einem zweiten Abstand voneinander angeordnet sind, und
die ersten und zweiten Abstände so gewählt sind, dass jeder der Drähte an der jeweiligen leitfähigen Stelle ausgerichtet ist.

5. Das Gerät gemäß Anspruch 1,
wobei das Führungselement ein Polymer umfasst.

6. Das Gerät gemäß Anspruch 1,
wobei das Führungselement rund um die Drähte geformt ist.

7. Das Gerät gemäß Anspruch 1,
wobei die Drähte aus jeweils acht Drähten bestehen.

8. Das Gerät gemäß Anspruch 1, wobei dieses zudem Folgendes umfasst:
ein weiteres Führungselement (390), das an die Leiterschnittstelle angrenzt, wobei das zusätzliche Führungselement mehrere mit der Bildgebungskonstruktion kommunizierende leitfähige Elemente (396) umfasst, und wobei die Drähte durch das zusätzliche Führungselement verlaufen, sodass jeder der Drähte mit dem jeweiligen leitfähigen Element in Kontakt steht.

9. Das Gerät gemäß Anspruch 8,
wobei das zusätzliche Führungselement mit dem Führungselement verbunden ist.

10. Das Gerät gemäß Anspruch 1, wobei dieses zudem Folgendes umfasst:
ein weiteres Führungselement, das mit der gegenüberliegenden Oberfläche der Leiterschnittstelle verbunden ist,
wobei die Drähte durch das Führungselement und das zusätzliche Führungselement verlaufen, und
wobei das Führungselement und das zusätzliche Führungselement gemeinsam die Drähte auf die Leiterschnittstelle ausrichten.

11. Das Gerät gemäß Anspruch 10,
wobei das zusätzliche Führungselement mehrere leitfähige, mit der Bildgebungskonstruktion verbundene Elemente umfasst, und wobei die Drähte durch das zusätzliche Führungselement verlaufen, sodass die einzelnen Drähte mit dem jeweils entsprechenden leitfähigen Element in Kontakt stehen.

12. Das Gerät gemäß Anspruch 10,
wobei das Führungselement mit dem zusätzlichen Führungselement verbunden ist.

13. Eine Methode zum Zusammensetzen eines intraluminalen Bildgebungsgeräts, wobei die Methode folgende Schritte umfasst:
Aufnehmen einer Bildgebungskonstruktion mit einer Leiterschnittstelle;
Aufnehmen mehrerer Drähten, die durch ein Führungselement verlaufen;
gemeinsames Ausrichten der Drähte auf die Leiterschnittstelle durch Verbinden einer Oberfläche des Führungselements mit einer gegenüberliegenden Oberfläche der Leiterschnittstelle unter Verwendung eines Klebstoffs; und
Verbinden der Drähte mit der Bildgebungskonstruktion an der Leiterschnittstelle.

14. Die Methode gemäß Anspruch 13,
wobei das Aufnehmen der durch das Führungselemente verlaufenden Drähte folgenden Schritt umfasst:
Formen des Führungselements rund um die Drähte.

15. Die Methode gemäß Anspruch 13,
wobei die Leiterschnittstelle mehrere mit der Bildgebungskonstruktion verbundene leitfähige Stellen umfasst, und wobei das Verbinden das Löten der einzelnen Drähte an eine entsprechende leitfähige Stelle umfass.

## Revendications

1. Dispositif d'imagerie intraluminale (102), comprenant :
un élément (115) allongé flexible conçu pour être positionné à l'intérieur d'un vaisseau d'un patient, ledit élément allongé flexible comprenant une partie proximale et une partie distale ;
un ensemble d'imagerie (110) disposé au niveau de la partie distale de l'élément allongé flexible, ledit ensemble d'imagerie comprenant une interface de conducteur (320) ;
une pluralité de fils (330) s'étendant le long d'une longueur de l'élément allongé flexible et en communication avec l'ensemble d'imagerie, dans lequel la pluralité de fils sont couplés à l'ensemble d'imagerie au niveau de l'interface de conducteur ; et
un élément de guidage (350) comprenant une surface couplée à une surface opposée de l'interface de conducteur au moyen d'un adhésif, dans lequel la pluralité de fils s'étendent à travers l'élément de guidage, et dans lequel l'élément de guidage aligne collectivement la pluralité de fils sur l'interface de conducteur.

2. Dispositif selon la revendication 1, dans lequel l'élément de guidage comprend une pluralité d'alésages (352), dans lequel chaque fil de la pluralité de fils s'étend à travers un alésage respectif de la pluralité d'alésages.

3. Dispositif selon la revendication 2, dans lequel l'interface de conducteur comprend une pluralité de plots conducteurs (322), et l'élément de guidage aligne chaque fil de la pluralité de fils avec un plot respectif de la pluralité de plots conducteurs.

4. Dispositif selon la revendication 3, dans lequel la pluralité de plots conducteurs sont espacés les uns des autres d'une première distance,
la pluralité d'alésages sont espacés les uns des autres d'une seconde distance, et
les première et seconde distances sont sélectionnées de telle sorte que chaque fil de la pluralité de fils est aligné avec le plot respectif de la pluralité de plots conducteurs.

5. Dispositif selon la revendication 1, dans lequel l'élément de guidage comprend un polymère.

6. Dispositif selon la revendication 1, dans lequel l'élément de guidage est moulé autour de la pluralité de fils.

7. Dispositif selon la revendication 1, dans lequel la pluralité de fils comprend huit fils.

8. Dispositif selon la revendication 1, comprenant en outre :
un autre élément de guidage (390) positionné adjacent à l'interface de conducteur, dans lequel ledit autre élément de guidage comprend une pluralité d'éléments conducteurs (396) en communication avec l'ensemble d'imagerie, dans lequel la pluralité de fils s'étend à travers ledit autre élément de guidage de telle sorte que chaque fil de la pluralité de fils entre en contact avec un élément conducteur respectif de la pluralité d'éléments conducteurs.

9. Dispositif selon la revendication 8, dans lequel l'autre élément de guidage est couplé à l'élément de guidage.

10. Dispositif selon la revendication 1, comprenant en outre :
un autre élément de guidage couplé à la surface opposée de l'interface de conducteur, dans lequel une pluralité de fils s'étendent à travers l'élément de guidage et l'autre élément de guidage, et dans lequel l'élément de guidage et l'autre élément de guidage alignent collectivement la pluralité de fils sur l'interface de conducteur.

11. Dispositif selon la revendication 10, dans lequel l'autre élément de guidage comprend une pluralité d'éléments conducteurs en communication avec l'ensemble d'imagerie, dans lequel la pluralité de fils s'étend à travers l'autre élément de guidage de telle sorte que chaque fil de la pluralité de fils entre en contact avec un élément conducteur respectif de la pluralité d'éléments conducteurs.

12. Dispositif selon la revendication 10, dans lequel l'élément de guidage est couplé à l'autre élément de guidage.

13. Procédé d'assemblage d'un dispositif d'imagerie intraluminale, ledit procédé comprenant :
l'obtention d'un ensemble d'imagerie comprenant une interface de conducteur ;
l'obtention d'une pluralité de fils s'étendant à travers un élément de guidage ;
l'alignement collectif de la pluralité de fils sur l'interface de conducteur part couplage d'une surface de l'élément de guidage à une surface opposée de l'interface de conducteur à l'aide d'un adhésif ; et
le couplage de la pluralité de fils à l'ensemble d'imagerie au niveau de l'interface de conducteur.

14. Procédé selon la revendication 13, dans lequel l'obtention de la pluralité de fils s'étendant à travers l'élément de guidage comprend :
la formation de l'élément de guidage autour de la pluralité de fils.

15. Procédé selon la revendication 13, dans lequel l'interface de conducteur comprend une pluralité de plots conducteurs en communication avec l'ensemble d'imagerie, et dans lequel le couplage comprend la soudure de chaque fil de la pluralité de fils à un plot conducteur respectif de la pluralité de plots conducteurs.
